# EUROPEAN PATENT SPECIFICATION

(11) **EP 3 799 840 B1**
(45) Date of publication and mention of the grant of the patent: **11.10.2023**
(21) Application number: 19892011.8
(22) Date of filing: 20.11.2019
(51) Int. Cl.: A61F 2/28, A61F 2/30

(54) **BONE TRABECULA STRUCTURE AND PROSTHESIS USING SAME AND MANUFACTURING METHOD THEREFOR**
KNOCHENTRABEKELSTRUKTUR UND PROTHESE MIT VERWENDUNG DAVON UND HERSTELLUNGSVERFAHREN DAFÜR
STRUCTURE DE TRABÉCULE OSSEUSE ET PROTHÈSE L'UTILISANT ET SON PROCÉDÉ DE FABRICATION

(30) Priority: 04.12.2018 CN 201811475455
(43) Date of publication of application: 07.04.2021
(73) Proprietor: Beijing Chunlizhengda Medical Instruments Co., Ltd, Beijing 101112 (CN)
(72) Inventor: SHI, Chunbao, Beijing 101112 (CN); XU, Kuixue, Beijing 101112 (CN); LU, Xiaoqiang, Beijing 101112 (CN); XIE, Fengbao, Beijing 101112 (CN); WANG, Zhenguo, Beijing 101112 (CN); DONG, Zeyu, Beijing 101112 (CN); SHI, Wenchao, Beijing 101112 (CN)
(74) Representative: Huang, Liwei
(86) International application number: PCT/CN2019/119619
(87) International publication number: WO 2020/114253

(56) References cited:
- WO-A1-2010/044758
- CN-A- 101 416 906
- CN-A- 101 980 214
- CN-A- 106 388 976
- CN-A- 106 388 976
- CN-A- 108 578 018
- CN-A- 109 481 092
- CN-U- 209 678 760
- US-A1- 2014 025 181
- US-A1- 2016 158 417
- US-A1- 2017 156 880
- US-A1- 2018 263 785
- RENDERS G. A. P. ET AL: "Porosity of human mandibular condylar bone", JOURNAL OF ANATOMY., vol. 210, no. 3, 1 March 2007 (2007-03-01) , pages 239-248, XP055807347, GB ISSN: 0021-8782, DOI: 10.1111/j.1469-7580.2007.00693.x

## Description

This application claims the priority benefit of China patent application No. 201811475455.3 titled "Bone Trabecular Structure And Prosthesis Having The Same" filed on December 4, 2018 by Beijing Chunlizhengda Medical Instruments Co., Ltd.

### FIELD OF THE INVENTION

The invention relates to a bone trabecular structure, a prosthesis and a fabrication method of the bone trabecular structure.

### DESCRIPTION OF RELATED ART

In a surgical operation, when tumor, nonunion after fracture or other pathological changes in the limb backbone of a human body lead to partial bone defects or surgical resections, it is necessary to replace them with an artificial prosthesis. In the operation of prosthesis replacement, an artificial prosthesis have to be connected and fused with bone tissues to restore the physiological function of the limb backbone.

At present, most of the existing prosthesis are cemented prosthesis and titanium-coated cementless prosthesis. Cemented prosthesis may suffer from a greater risk of prosthesis loosening in the later stage, which will lead to surgical failure. However, titanium-coated prosthesis will produce metal corrosion in complex human body environment, resulting in the release of toxic elements and thus reduced biocompatibility. In addition, the elastic modulus of the metal material is quite different from that of human bone tissues, which is easy to produce stress shielding effect, which is not conducive to the growth and remodeling of new bones, and even leads to secondary fracture. Moreover, the repair of bone defects caused by bone trauma and necrosis has poor mechanical and osteoinductive properties.

In order to improve the bone ingrowth effect after prosthesis implantation, a bone trabecular structure is increasingly applied to an artificial prosthesis in the field. The bone trabecular structure is mainly used to make the prosthesis form a number of pores conducive to bone ingrowth, so as to improve the stability of the prosthesis after implantation. However, the existing bone trabecular structure usually has uniform and equal diameter distribution, and lacks bionic characteristics of the real structure of a human body. In the actual service process after implantation, the structural strength of some parts is insufficient due to the uneven stress characteristics of the human body, while the structural strength of other parts suffers from over designing. If the bone trabecular structure is not in the best condition with the joint face of the bone, the early stability of the prosthesis will be affected, and then the growth of the bone will be affected, which is not conducive to long-term and stable prosthesis service.

WO 2010/044758 A1 discloses bioresorbable scaffolds for bone engineering, such as repair of bone defects, particularly long bone defects, or augmentation of bone length are described. Scaffolds are porous and comprise multiple side channels. In one embodiment, scaffolds are made from layers of micro-filament meshes comprising polycaprolactone (PCL) or a PCL-composite sequentially laid in incremental 60 degrees of rotation to produce a 0/60/120 degree layering pattern, providing for the formation of interconnected pores.

In view of the deficiencies present in the prior art, those skilled in the art urgently hope to seek a bone trabecular structure which is more conducive to bone ingrowth and improve the postoperative recovery effect, so as to address the deficiencies present in the prior art.

Patent document US 2014/025181 A1 discloses a fabrication method of a bone trabecular structure comprising the following steps:Step 1: scanning a natural bone trabecular structure by a Micro CT and remodeling the scanned data to obtain a three-dimensional schematic model of the bone trabecular structure, so as to obtain a basic structure model of the bone trabecular structure in advance; Step 2: adjusting the diameter of the struts in the three-dimensional schematic model of the bone trabecular structure to be at the desired range, and adjusting the diameter of the pores formed by the struts so that the average diameter of the pores ranges as desired, and the porosity of the three-dimensional schematic model ranges as desired; Step 3: dividing the three-dimensional schematic model of the bone trabecular structure into different regions which are respectively adapted for different growth requirements of the same tissue, and further adjusting the diameters of the pores in the regions so that different regions have different porosities, whereby a bone tissue can grow into the bone trabecular structure more quickly and adaptively under different bone growth requirements; and Step 4: generating a solid model of the bone trabecular structure by using a 3D printing equipment.

### BRIEF SUMMARY OF THE INVENTION

In order to facilitate bone ingrowth and improve the postoperative recovery effect of a patient, the present application provides a bone trabecular structure, a prosthesis having the same and a fabrication method thereof.

The invention is set out in the appended set of claims.

According to a first aspect of the present application, a bone trabecular structure is provided, which includes a body configured to be a three-dimensional porous structure. The three-dimensional porous structure includes a plurality of struts and a plurality of pores formed by staggered connection of the plurality of struts. The pores are communicated with each other, and the average diameters of the pores are different from each other. In particular, the average diameter of the pores ranges from 100 µm to 400 µm, and the porosity of the three-dimensional porous structure ranges from 50% to 80%. The three-dimensional structure includes a plurality of regions adapted to different growth requirements of the same tissue, and the porosity of the regions is different from each other. The pores (12) have different average diameters. The three-dimensional structure (1) comprises at least one region in which the density of pores (12) in the direction from the outside to the inside of the three-dimensional porous structure (1) increases gradually.

The diameter of the struts ranges from 100 µm to 200 µm.

Further, the cross-section shape of the pores is irregular polygon.

Further, a plurality of convex portions are formed on the peripheral walls of the struts.

Further, the three-dimensional porous structure is made of titanium alloy material.

Further, the elastic modulus of the three-dimensional porous structure ranges from 5-30 GPa.

Further, the maximum static friction coefficient of the outer surface of the three-dimensional porous structure ranges from 1.2 to 1.5.

According to a second aspect of the present application, a prosthesis is provided. The prosthesis includes a prosthesis body and the above bone trabecular structure formed on an outer surface of the prosthesis body.

According to a third aspect of the present application, a fabrication method of a bone trabecular structure is provided, which includes the following steps:
Step 1: scanning a natural bone trabecular structure by a Micro CT, and remodeling the scanned data by using MIMICS to obtain a three-dimensional schematic model of the bone trabecular structure. The Step 1 is used for obtaining the basic structure model of the bone trabecular structure in advance;
Step 2: adjusting the diameter of the struts in the three-dimensional schematic model of the bone trabecular structure to be between 100 µm and 200 µm, and adjusting the diameter of the pores formed by the struts so that the average diameter range of the pores ranges from 100 µm to 400 µm, and the porosity of the three-dimensional schematic model ranges from 50% to 80%;
Step 3: dividing the three-dimensional schematic model of the bone trabecular structure into different regions which are respectively adapted for different growth requirements of the same tissue, wherein the porosities of the divided regions are different from each other, and the different regions comprise at least one region in which the density of pores (12) in the direction from the outside to the inside increases gradually, and further adjusting the diameter of the pores in the regions so that different regions have different porosity, whereby a bone tissue can grow into the bone trabecular structure more quickly and adaptively under different bone growth requirements;
Step 4: generating a solid model of the bone trabecular structure by using a 3D printing equipment, in which the focus offset parameter of the 3D printing device is adjusted so that a plurality of bumps are formed on the surface of the struts in the generated solid model. The value of the focus offset parameter ranges from 5.8 mA to 6.2 mA.

Further, the cross-section shape of the pores is irregular polygon.

Further, a plurality of convex portions are formed on the peripheral wall of the struts.

Further, the struts and the pores form a three-dimensional porous structure which is made of titanium alloy material.

Further, the elastic modulus of the three-dimensional structure ranges from 5 to 30 GPa.

Further, the maximum static friction coefficient of the outer surface of the three-dimensional porous structure ranges from 1.2 to 1.5.

In the bone trabecular structure of the present application, the pores formed by staggered connection of the struts are communicated with each other, the average diameter of the pores is configured to be different from each other, and the average diameter and porosity of the pores of the three-dimensional porous structure are specifically set, so that the structure of the bone trabecular structure more resembles that of the bone trabecular structure in a human body, and the bone of the human body can grow into the pores of the three-dimensional porous structure rapidly and naturally, and facilitate a quick postoperative fusion and fixation of the bone trabecular structure with the bone tissues of the human body. Therefore, it can effectively improve the postoperative recovery effect of a patient.

### BRIEF DESCRIPTION OF DRAWINGS

FIG. 1 is an enlarged perspective view of part of the trabecular structure according to the present application;
FIG. 2 is a schematic diagram of the main cross section of the trabecular structure shown in FIG. 1; and
FIG. 3 is a flow chart of the fabrication method of the bone trabecular structure according to the present application.

### DETAILED DESCRIPTION

Embodiments of the technical solutions of the present application will be described in detail in connection with the drawings. The following embodiments are merely provided for explaining the technical solutions of the present application more clearly, and thus is only used as an example, without any intention to limit the protection scope of the present application.

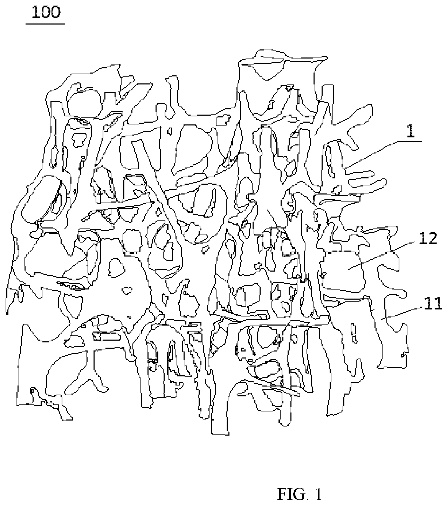

FIG. 1 shows the structure of a bone trabecular structure 100 according to the present application. The bone trabecular structure 100 includes a body configured to be a three-dimensional porous structure 1. The three-dimensional porous structure 1 includes a plurality of struts 11 and a plurality of pores 12 formed by staggered connection of the plurality of struts 11. The pores 12 are communicated with each other, and the average diameter of the pores 12 is different from each other. In particular, the average diameter of the pores 12 ranges from 100 µm to 400 µm, and the porosity of the three-dimensional porous structure ranges from 50% to 80%.

In the bone trabecular structure 100 of the present application, the pores 12 formed by staggered connection of the struts 11 are communicated with each other, the average diameter of the pores 12 is configured to be different from each other, and the average diameter and porosity of the pores 12 of the three-dimensional porous structure 1 are specifically set, so that the structure of the bone trabecular structure 100 more resembles that of the bone trabecular structure in a human body, and the bone of the human body can grow into the pores 12 of the three-dimensional porous structure 1 rapidly and naturally, and facilitate a quick postoperative fusion and fixation of the bone trabecular structure 100 with the bone tissues of the human body. Therefore, it can effectively improve the postoperative recovery effect of a patient.

The three-dimensional porous structure 1 includes a plurality of regions adapted to different growth requirements of the same tissue, and the porosity of individual regions is different from each other. That is, in different regions of the three-dimensional porous structure 1, regarding different bone ingrowth requirements, the porosity of different regions can be set specifically. The porosity of different regions can be set by controlling the average diameter of the pores in corresponding regions. For example, when some region of the bone trabecular structure 100 needs to contact with bone, it is preferred to set the average diameter range of pores in this region between 200 µm and 400 µm, and further preferably between 300 µm and 400 µm in order to achieve rapid bone ingrowth for which the porosity in this region can be controlled between 50% and 60%. When some region of the bone trabecular structure 100 does not need to contact with bone, but needs to facilitate bone crawling, the range of average diameter of pores in this region can be set between 100 µm and 200 µm for which the porosity of this region can be controlled between 70% and 75%. When some region of the trabecular structure 100 neither needs to contact with bone nor needs to facilitate bone crawling, the average diameter range of pores in this region can be set between 100 µm and 150 µm for which the porosity of this region can be controlled between 75% and 80%.

In the same region, the density of the pores 12 in the direction from the outside to the inside of the three-dimensional porous structure 1 gradually increases, so that the bone tissues can grow more smoothly and quickly from the outside of the three-dimensional porous structure 1 into the internal center of the three-dimensional porous structure 1, so as to improve the fusion and fixation of the bone trabecular structure 100 with human bone tissues.

Further preferably, in order to ensure the structural strength of the three-dimensional porous structure 1, the diameter range of the struts 11 can be set between 100 µm and 200 µm. Preferably, the diameter range of the struts 11 is set between 150 µm and 200 µm, and further preferably, the diameter of the struts 11 is 180 µm.

As shown in Fig. 1, the cross-sectional shape of the pores 12 of the three-dimensional porous structure 1 is irregular polygon, and its particular shape can be roughly tetrahedron or hexahedron.

In a preferred embodiment, a plurality of convex portions (not shown) can be formed on the peripheral wall of the struts 11. The providing of the plurality of convex portions is used to increase the roughness of the peripheral wall of the struts 11, so as to increase the friction force of the peripheral wall. In this way, the fixation of bone tissues with strut 11 is more firm and stable during bone ingrowth.

Preferably, the convex portion can be a circular convex point or a conical convex point. It should be noted that the shape of the convex portion is not limited to the above-mentioned shapes, as long as the convex portion can effectively increase the external surface area of the struts 11 to increase the friction force of its peripheral wall, and thus will not be described here in detail. Further, the width range of the maximum profile shape of the cross section of the convex portion is 5-50 µm and the maximum height range is 10-50 µm.

Further preferably, the convex portions can be evenly distributed on the outer surface of the struts 11, or can be distributed in a discrete manner. Further preferably, the number of convex portions of the struts 11 in a region of the bone trabecular structure 100 having a higher porosity may be greater than the number of the convex portions of the struts 11 in a region of the bone trabecular structure 100 having a lower porosity, so as to facilitate firmer bone ingrowth. It is further preferred that the density of the convex portions on the struts 11 at an outer edge of the three-dimensional porous structure 1 is greater than that of the convex portions on the struts 11 at the interior of the three-dimensional porous structure 1.

According to the present application, the three-dimensional porous structure 1 can be made of metal powder, which can be titanium alloy, pure titanium or tantalum metal, etc. Preferably, the three-dimensional porous structure 1 is made of titanium alloy material, preferably made of Ti6Al4V.

According to the present application, the elastic modulus of the three-dimensional porous structure 1 ranges from 5 to 30 GPa. This range of the elastic modulus can provide the bone trabecular structure 100 with better mechanical properties, that is, better compression and torsion resistance, so as to ensure the stability of the bone trabecular structure 100 after implantation into a human body.

Further, the maximum static friction coefficient of the outer surface of the three-dimensional porous structure 1 ranges from 1.2 to 1.5. In this range, the external surface of the bone trabecular structure 100 has a roughness more suitable for bone growth or crawling, thus facilitating further improving the speed and stability of bone ingrowth therein. Meanwhile, compared with a bone trabecular structure in the prior art, the maximum static friction force of the external surface of the bone trabecular structure 1 in the present application is far greater than that of the bone trabecular structure in the prior art, so that the bone trabecular structure 100 of the present application significantly differs from those in the prior art, and the bone trabecular structure 100 of the present application provide more suitable conditions for human bone growth and bone crawling. Upon testing, it is shown that, under the same testing conditions, the maximum static friction coefficient of the bone trabecular structure 100 of the present application is 1.35 under unit pressure, while the maximum static friction coefficient of the bone trabecular structure 100 in the prior art under unit pressure is only 1.08.

In addition, the present application provides a prosthesis (not shown). The prosthesis includes a prosthesis body and the above-mentioned bone trabecular structure 100 formed on the outer surface of the prosthesis body. Since the bone trabecular structure 100 of the present application more resembles the bone trabecular structure of a human body, the bone of the human body can quickly and naturally grow into the pores 12 of the three-dimensional porous structure 1, which is conducive to a rapid fusion and fixation of the prosthesis with the bone tissues of the human body after an operation, thereby effectively improving the postoperative recovery effect of a patient.

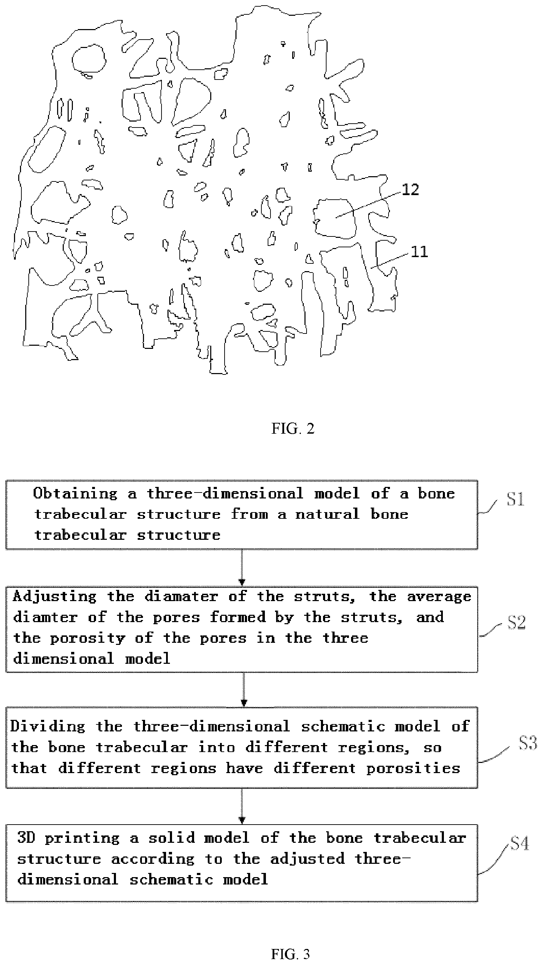

Fig. 3 shows a flow chart of a fabrication method of a bone trabecular structure according to the present application. As shown in FIG. 3, the fabrication method of the bone trabecular structure includes the following steps:
Step 1: scanning a natural bone trabecular structure by a Micro CT, and remodeling the scanned data by using MIMICS to obtain a three-dimensional schematic model of the bone trabecular structure. This step is used for obtaining the basic structure model of the bone trabecular structure in advance.
Step 2: adjusting the diameter of the struts 11 in the three-dimensional schematic model of the bone trabecular structure to be between 100 µm and 200 µm, and adjusting the diameter of the pores 12 formed by the struts 11 so that the average diameter of the pores 12 ranges from 100 µm to 400 µm, and the porosity of the three-dimensional schematic model ranges from 50% to 80%.
Step 3: dividing the three-dimensional schematic model of the bone trabecular structure into different regions which are respectively adapted for different growth requirements of the same tissue, and further adjusting the diameter of the pores 12 in the regions so that different regions have different porosity, whereby a bone tissue can grow into the bone trabecular structure more quickly and adaptively under different bone growth requirements.
Step 4: generating a solid model of the bone trabecular structure 100 by using a 3D printing equipment, in which the focus offset parameter of the 3D printing device is adjusted so that a plurality of bumps are formed on the surface of the struts 11 in the generated solid model. In particular, the value of the focus offset parameter ranges from 5.8 mA to 6.2 mA.

Upon testing, it is shown that the bone trabecular structure 100 of the present application has good initial stability and good bone ingrowth performance, that is, the growing-in depth of the bone can reach 60% to 80%. The prosthesis using the bone trabecular structure 100 of the present application has a very stable effect after implantation, effectively reduces the postoperative recovery time of a patient, and improves the postoperative recovery effect of the patient.

It should be noted that, unless otherwise specified, the technical terms or scientific terms used in the present application shall have the common meaning understood by those skilled in the art.

Finally, it should be noted that, the above embodiments are merely provided for illustrating the technical solutions of the present application, without any limitation thereto. Although the invention has been described in detail with reference to the above-mentioned embodiments, those skilled in the art will understand that, the technical solutions recited in the above-mentioned embodiments can still be modified or some or all of the technical features therein can be equivalently substituted by those skilled in the art. In particular, as long as there is no structural conflict, the technical features mentioned in individual embodiments can be combined in any way. The invention is not limited to the specific embodiments disclosed herein, but includes all technical solutions falling within the scope of the claims.

## Claims

1. A bone trabecular structure (100), comprising a body configured to be a three-dimensional porous structure (1) comprising a plurality of struts (11) and a plurality of pores (12) formed by staggered connection of the plurality of struts (11), wherein the pores (12) are communicated with each other, wherein average diameters of the pores (12) range from 100 µm to 400 µm, and the porosity of the three-dimensional porous structure (1) ranges from 50% to 80%, **characterized in that**
the three-dimensional structure (1) comprises a plurality of regions adapted to different growth requirements of the same tissue, and the porosities of the regions are different from each other, and the pores (12) have different average diameters, wherein the three-dimensional structure (1) comprises at least one region in which the density of pores (12) in the direction from the outside to the inside of the three-dimensional porous structure (1) increases gradually.

2. The bone trabecular structure according to claim 1, wherein the diameter of the struts (11) ranges from 100 µm to 200 µm.

3. The bone trabecular structure according to claim 1, wherein the cross-section shape of the pores (12) is irregular polygon.

4. The bone trabecular structure according to any one of claims 1-3, wherein a plurality of convex portions are formed on the peripheral walls of the struts (11).

5. The bone trabecular structure according to any one of claims 1-3, wherein the three-dimensional porous structure (1) is made of titanium alloy material.

6. The bone trabecular structure according to claim 5, wherein the elastic modulus of the three-dimensional porous structure (1) ranges from 5-30 GPa.

7. A prosthesis **characterized by** comprising a prosthesis body and the bone trabecular structure (100) according to any one of claims 1-6 formed on an outer surface of the prosthesis body.

8. A fabrication method of a bone trabecular structure (100) according to any of the previous claims, the method comprising the following steps:
Step 1: scanning a natural bone trabecular structure by a Micro CT, and remodeling the scanned data by using MIMICS to obtain a three-dimensional schematic model of the bone trabecular structure (100), so as to obtain a basic structure model of the bone trabecular structure (100) in advance;
Step 2: adjusting the diameter of the struts (11) in the three-dimensional schematic model of the bone trabecular structure (100) to be between 100 µm and 200 µm, and adjusting the diameter of the pores (12) formed by the struts (11) so that average diameters of the pores (12) range from 100 µm to 400 µm, and the porosity of the three-dimensional schematic model ranges from 50% to 80%;
Step 3: dividing the three-dimensional schematic model of the bone trabecular structure (100) into different regions which are respectively adapted for different growth requirements of the same tissue, wherein the porosities of the divided regions are different from each other, and the different regions comprise at least one region in which the density of pores (12) in the direction from the outside to the inside increases gradually, and further adjusting the diameters of the pores (12) in the regions so that different regions have different porosities, whereby a bone tissue can grow into the bone trabecular structure (100) more quickly and adaptively under different bone growth requirements; and
Step 4: generating a solid model of the bone trabecular structure (100) by using a 3D printing equipment, in which the focus offset parameter of the 3D printing device is adjusted, so that a plurality of bumps are formed on the surface of the struts (11) in the generated solid model, wherein the value of the focus offset parameter ranges from 5.8 mA to 6.2 mA.

9. The fabrication method of a bone trabecular structure (100) according to claim 8, wherein the cross-section shape of the pores (12) is irregular polygon.

10. The fabrication method of a bone trabecular structure (100) according to claim 8, wherein a plurality of convex portions are formed on the peripheral wall of the struts (11).

11. The fabrication method of a bone trabecular structure (100) according to claim 8, wherein the struts (11) and the pores (11) form a three-dimensional porous structure (1) which is made of titanium alloy material.

## Patentansprüche

1. Knochentrabekelstruktur (100), umfassend einen Körper, der als dreidimensionale poröse Struktur (1) konfiguriert ist, die eine Vielzahl von Streben (11) und eine Vielzahl von Poren (12) umfasst, die durch eine versetzte Verbindung der Vielzahl von Streben (11) gebildet ist, wobei die Poren (12) miteinander in Verbindung stehen, wobei die durchschnittlichen Durchmesser der Poren (12) im Bereich von 100 µm bis 400 µm liegen und die Porosität der dreidimensionalen porösen Struktur (1) im Bereich von 50 % bis 80 % liegt, **dadurch gekennzeichnet, dass**
die dreidimensionale Struktur (1) eine Vielzahl von Regionen umfasst, die an unterschiedliche Wachstumsanforderungen desselben Gewebes angepasst ist, und sich die Porositäten der Regionen voneinander unterscheiden und die Poren (12) unterschiedliche durchschnittliche Durchmesser aufweisen, wobei die dreidimensionale Struktur (1) mindestens eine Region umfasst, in der die Dichte der Poren (12) in Richtung von außerhalb nach innerhalb der dreidimensionalen porösen Struktur (1) allmählich zunimmt.

2. Knochentrabekelstruktur nach Anspruch 1, wobei der Durchmesser der Streben (11) im Bereich von 100 µm bis 200 µm liegt.

3. Knochentrabekelstruktur nach Anspruch 1, wobei die Querschnittsform der Poren (12) ein unregelmäßiges Polygon ist.

4. Knochentrabekelstruktur nach einem der Ansprüche 1-3, wobei an den Umfangswänden der Streben (11) eine Vielzahl von konvexen Abschnitten gebildet ist.

5. Knochentrabekelstruktur nach einem der Ansprüche 1-3, wobei die dreidimensionale poröse Struktur (1) aus einem Titanlegierungsmaterial besteht.

6. Knochentrabekelstruktur nach Anspruch 5, wobei der Elastizitätsmodul der dreidimensionalen porösen Struktur (1) im Bereich von 5-30 GPa liegt.

7. Prothese, **dadurch gekennzeichnet, dass** sie einen Prothesenkörper und die Knochentrabekelstruktur (100) nach einem der Ansprüche 1-6 umfasst, die auf einer Außenfläche des Prothesenkörpers gebildet ist.

8. Fertigungsverfahren einer Knochentrabekelstruktur (100) nach einem der vorhergehenden Ansprüche, wobei das Verfahren die folgenden Schritte umfasst:
Schritt 1: Scannen einer natürlichen Knochentrabekelstruktur mit einem Mikro-CT und Remodellieren der gescannten Daten unter Verwendung von MIMICS, um ein dreidimensionales schematisches Modell der Knochentrabekelstruktur (100) zu erhalten, um somit ein Grundstrukturmodell der Knochentrabekelstruktur (100) im Voraus zu erhalten;
Schritt 2: Einstellen des Durchmessers der Streben (11) in dem dreidimensionalen schematischen Modell der Knochentrabekelstruktur (100) auf zwischen 100 µm und 200 µm und Einstellen des Durchmessers der durch die Streben (11) gebildeten Poren (12), sodass die durchschnittlichen Durchmesser der Poren (12) im Bereich von 100 µm bis 400 µm liegen und die Porosität des dreidimensionalen schematischen Modells im Bereich von 50 % bis 80 % liegt;
Schritt 3: Aufteilen des dreidimensionalen schematischen Modells der Knochentrabekelstruktur (100) in unterschiedliche Regionen, die jeweils an unterschiedliche Wachstumsanforderungen desselben Gewebes angepasst sind, wobei sich die Porositäten der unterteilten Regionen voneinander unterscheiden und die unterschiedlichen Regionen mindestens eine Region umfassen, in der die Dichte der Poren (12) in der Richtung von außerhalb nach innerhalb allmählich zunimmt, und ferner Einstellen der Durchmesser der Poren (12) in den Regionen, sodass unterschiedliche Bereiche unterschiedliche Porositäten aufweisen, wodurch ein Knochengewebe bei unterschiedlichen Knochenwachstumsanforderungen schneller und anpassungsfähiger in die Knochentrabekelstruktur (100) hineinwachsen kann; und
Schritt 4: Generieren eines Volumenmodells der Knochentrabekelstruktur (100) unter Verwendung einer 3D-Druckausrüstung, bei welcher der Fokusversatzparameter der 3D-Druckvorrichtung so eingestellt wird, dass in dem generierten Volumenmodell auf der Oberfläche der Streben (11) eine Vielzahl von Erhebungen gebildet wird, wobei der Wert des Fokus-Offset-Parameters im Bereich von 5,8 mA bis 6,2 mA liegt.

9. Fertigungsverfahren einer Knochentrabekelstruktur (100) nach Anspruch 8, wobei die Querschnittsform der Poren (12) ein unregelmäßiges Polygon ist.

10. Fertigungsverfahren einer Knochentrabekelstruktur (100) nach Anspruch 8, wobei eine Vielzahl von konvexen Abschnitten an der Umfangswand der Streben (11) gebildet ist.

11. Fertigungsverfahren einer Knochentrabekelstruktur (100) nach Anspruch 8, wobei die Streben (11) und die Poren (11) eine dreidimensionale poröse Struktur (1) bilden, die aus einem Titanlegierungsmaterial besteht.

## Revendications

1. Structure de trabécule osseuse (100), comprenant un corps conçu pour être une structure poreuse tridimensionnelle (1) comprenant une pluralité d'entretoises (11) et une pluralité de pores (12) formés par un raccordement en quinconce de la pluralité d'entretoises (11), lesdits pores (12) étant en communication les uns avec les autres, les diamètres moyens des pores (12) allant de 100 µm à 400 µm, et la porosité de la structure poreuse tridimensionnelle (1) allant de 50 % à 80 %,
**caractérisée en ce que**
la structure tridimensionnelle (1) comprend une pluralité de zones adaptées à différentes exigences de croissance du même tissu, et les porosités des zones sont différentes les unes des autres, et les pores (12) comportent des diamètres moyens différents, ladite structure tridimensionnelle (1) comprenant au moins une zone dans laquelle la densité de pores (12) dans la direction de l'extérieur vers l'intérieur de la structure poreuse tridimensionnelle (1) augmente progressivement.

2. Structure de trabécule osseuse selon la revendication 1, le diamètre des entretoises (11) étant compris entre 100 µm à 200 µm.

3. Structure de trabécule osseuse selon la revendication 1, la forme en coupe transversale des pores (12) étant un polygone irrégulier.

4. Structure de trabécule osseuse selon l'une quelconque des revendications 1 à 3, une pluralité de parties convexes étant formées sur les parois périphériques des entretoises (11).

5. Structure de trabécule osseuse selon l'une quelconque des revendications 1 à 3, ladite structure poreuse tridimensionnelle (1) étant constituée d'un matériau en alliage de titane.

6. Structure de trabécule osseuse selon la revendication 5, ledit module d'élasticité de la structure poreuse tridimensionnelle (1) allant de 5 à 30 GPa.

7. Prothèse **caractérisée en ce qu'**elle comprend un corps de prothèse et la structure de trabécule osseuse (100) selon l'une quelconque des revendications 1 à 6 formée sur une surface externe du corps de prothèse.

8. Procédé de fabrication d'une structure de trabécule osseuse (100) selon l'une quelconque des revendications précédentes, le procédé comprenant les étapes suivantes :
Étape 1 : balayage d'une structure de trabécule osseuse naturelle par une micro CT, et remodélisation des données scannées à l'aide de MIMICS pour obtenir un modèle schématique tridimensionnel de la structure de trabécule osseuse (100), afin d'obtenir un modèle de structure de base de la structure de trabécule osseuse (100) à l'avance ;
Étape 2 : ajustement du diamètre des entretoises (11) dans le modèle schématique tridimensionnel de la structure de trabécule osseuse (100) pour qu'ils soient compris entre 100 µm et 200 µm, et ajustement du diamètre des pores (12) formés par les entretoises (11) de sorte que les diamètres moyens des pores (12) aillent de 100 µm à 400 µm, et que la porosité du modèle schématique tridimensionnel aille de 50 % à 80 % ;
Étape 3 : division du modèle schématique tridimensionnel de la structure de trabécule osseuse (100) en différentes zones qui sont respectivement adaptées aux différentes exigences de croissance du même tissu, lesdites porosités des zones divisées étant différentes les unes des autres, et lesdites différentes zones comprenant au moins une zone dans laquelle la densité des pores (12) dans la direction de l'extérieur vers l'intérieur augmente progressivement, et ajustement en outre des diamètres des pores (12) dans les zones de sorte que des zones différentes comportent des porosités différentes, moyennant quoi un tissu osseux peut se développer dans la structure de trabécule osseuse (100) plus rapidement et de manière adaptative en vertu des exigences de croissance osseuse différentes ; et
Étape 4 : génération d'un modèle solide de la structure de trabécule osseuse (100) à l'aide d'un équipement d'impression 3D, le paramètre de décalage de focalisation du dispositif d'impression 3D étant ajusté de sorte qu'une pluralité de bosses soient formées à la surface des entretoises (11) dans le modèle solide généré, la valeur du paramètre de décalage de focalisation allant de 5,8 mA à 6,2 mA.

9. Procédé de fabrication d'une structure de trabécule osseuse (100) selon la revendication 8, ladite forme en coupe transversale des pores (12) étant un polygone irrégulier.

10. Procédé de fabrication d'une structure de trabécule osseuse (100) selon la revendication 8, une pluralité de parties convexes étant formées sur la paroi périphérique des entretoises (11).

11. Procédé de fabrication d'une structure de trabécule osseuse (100) selon la revendication 8, lesdites entretoises (11) et lesdits pores (11) formant une structure poreuse tridimensionnelle (1) qui est constituée d'un matériau en alliage de titane.
